# EUROPEAN PATENT APPLICATION

(11) **EP 4 787 106 A1**
(43) Date of publication of application: **05.08.2026**
(21) Application number: 25155795.5
(22) Date of filing: 04.02.2025
(51) Int. Cl.: G05D 1/247, G05D 1/686, G05D 105/30, G05D 107/60, G05D 109/10, G05D 111/20, A61B 6/00

(54) **MOBILE RADIOGRAPHY APPARATUS**

(71) Applicant: Koninklijke Philips N.V., 5656 AE Eindhoven (NL)
(72) Inventor: DIXIT, Deepak Dilipsingh, Eindhoven (NL); AGRAHARI, Amit, Eindhoven (NL); SAMANTA, Sohham, 5656AG Eindhoven (NL); KUMAR, Rakesh, Eindhoven (NL)
(74) Representative: Philips Intellectual Property & Standards

(57) **Abstract**

The subject-matter of the present disclosure relates to a mobile radiography apparatus (100), comprising a first set of sensors (116), comprising at least one sensor, configured to receive a signal emitted by a beacon device (200), a controller (118) configured control a linear movement of the mobile radiography apparatus based on the received signal, a second set of sensors (120), comprising at least one sensor, configured to detect a position of the beacon device, wherein the controller is configured to control a rotational movement of the mobile radiography apparatus to adjust an alignment of the mobile radiography apparatus with respect to the beacon device, based on the detected position of the beacon device.

## Description

### FIELD OF THE INVENTION

The subject-matter of the present disclosure relates to a mobile radiography apparatus. In particular, the disclosure relates to a mobile radiography apparatus with automatic navigation; yet more specifically a mobile radiography apparatus with `follow me' functionality.

### BACKGROUND OF THE INVENTION

In the demanding environment of modern hospitals, the transportation of mobile x-ray systems presents a significant challenge. These systems, comprising an x-ray tube, generator, and a variety of supporting mechanical and electrical components, are inherently heavy and cumbersome. Technicians tasked with moving these devices face considerable physical strain and risk of injury, particularly when navigating through crowded and complex hospital layouts.

Manual transportation consumes valuable time, which could be better utilized in patient care. Furthermore, the risk of collision with hospital infrastructure or other equipment poses a safety hazard not only to the technicians but also to patients and staff.

These challenges highlight the need for a more efficient, user-friendly, and safe method of mobilizing x-ray systems within hospital settings. Addressing these issues is crucial for improving overall workflow, reducing physical stress on technicians, and enhancing patient care quality.

In recent times automatic/powered movement of mobile medical devices have been proposed, such as remote controlled devices or those that navigate to a received signal. However prior art approaches are not always suitable for heavy radiography equipment.

It is therefore an aim of the subject-matter of the present disclosure to improve on the prior art.

### SUMMARY OF THE INVENTION

According to a first aspect of the present invention, there is provided a mobile radiography apparatus, comprising a first set of sensors, comprising at least one sensor, configured to receive a signal emitted by a beacon device, a controller configured control a linear movement of the mobile radiography apparatus based on the received signal, a second set of sensors, comprising at least one sensor, configured to detect a position of the beacon device, wherein the controller is configured to control a rotational movement of the mobile radiography apparatus to adjust an alignment of the mobile radiography apparatus with respect to the beacon device, based on the detected position of the beacon device.

In this way, the apparatus provides an advantage over prior art systems which may only rely on navigation by following UWB tag (or similar tag). The present approach overcomes any issues with wobble, navigation deviation, etc, that are more likely to arise from a heavy mobile radiographic imaging apparatus.

In an example, the first set of sensors are configured to receive ultra-wide band radio signals.

In an example, the first set of sensors comprises at least three sensors.

In an example, the first set of sensors comprises at least four sensors, wherein two of the at least four sensors are arranged towards a front of the mobile radiography apparatus, and two the at least four sensors are arranged towards a rear of the mobile radiography apparatus.

In an example, the controller is configured to, based on not detecting the beacon device by the second set of sensors, determine a distance to the beacon device from each of the first set of sensors, and control the movement of the mobile radiography apparatus to orientate a forward face of the mobile radiography apparatus towards the beacon device.

In an example, the second set of sensors are configured to detect ultra sound.

In an example, detecting the position of the beacon device comprises detecting a position of an operator carrying the beacon device.

In an example, detecting the position of the beacon device is based on dividing a detection area of the second set of sensors into zones, and adjusting the alignment of the mobile radiography apparatus is based on the zone in which the locator beacon is detected.

In an example, each of the second set of sensors is arranged with a field of view pointing forward of the mobile radiography apparatus. In an example, separate zones are defined by separate fields of view corresponding to the second set of sensors. In an example, detecting the position of the beacon device is based on detecting the beacon device within the field of view.

In an example, second set of sensors comprises at least three sensors.

In a related aspect of the present invention, there is provided a mobile radiography system comprising a remote controller comprising a beacon device and the aforementioned mobile radiography apparatus.

In an example, the remote controller comprises at least one actuator, and movement of the actuator controls a movement of the mobile radiography apparatus.

In an example, the beacon device is configured to emit an ultra-wide band radio signal. These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
- Fig. 1: shows an example mobile radiography apparatus;
- Fig. 2: shows an example mobile radiography system travelling in a forward direction; and
- Fig. 3: shoes an example mobile radiography system initiating a turn.

### DETAILED DESCRIPTION OF EMBODIMENTS

Ultra-wideband (UWB), is a short-range radiofrequency (RF) technology for wireless communication that can be leveraged to detect the location of people, devices, and assets with high precision. Like other communication protocols including Bluetooth and Wi-Fi, UWB can be used to transmit data between devices through radio waves. It does so with short nanosecond pulses over an "ultra-wide" range of frequencies.

It can transmit very high data rates over short ranges, and pinpoint an exact location in real-time. UWB operates with a high bandwidth over a very wide frequency spectrum between 3.1 to 10.6 GHz. It also consumes very little power, allowing for affordable and efficient hardware options, such as tracking tags with coin cell batteries that can operate for multiple years without being recharged or replaced.

UWB detects location based on distance-based measurement via time-of-flight (ToF), that calculates location based on how long it takes for pulses of radio to travel from one device to another. In many scenarios, the location of UWB signals can be determined with an accuracy of less than 50 centimeters (with optimal conditions and deployment), and extremely low latency.

There are two primary approaches to utilising time of flight to determine position via UWB signals: Time Difference of Arrival (TDoA) and Two-Way Ranging (TWR).

TDoA utilizes UWB anchors or sensors that are deployed in a fixed position throughout an indoor space. These sensors then detect and locate a transmitting UWB device, such as a tracking tag. Suitably the fixed anchors need to be accurately synchronized to run on the same clock. The UWB tag, or other device, will transmit signals in regular intervals. These signals will be received by any anchors in the communication range and time-stamped by the anchors. All the time-stamped data is then sent to a central controller for analysis.

A location engine will analyse each anchor's data and the differences in arrival times to each anchor and use multilateration to calculate the tag's coordinates. Those coordinates can be used to visualize the location of the device on an indoor map of a space or leveraged for other uses depending on the specific application.

On the other hand, TWR primarily uses two-way communication between two devices to sense the distance between them. With TWR, when a device is in close proximity to another, the two devices will start ranging with each other to determine their distance, even as they communicate. The time it takes a signal to travel between them is then multiplied by the speed of light and used to determine their relative positions, frequently to enable location-aware communication.

The determined location from one device to another is then harnessed depending on the specific application. TWR can also be used by fixed anchors and UWB devices, however the TWR process can only use one ranging partner to locate the device at a time.

Other RF communications such as Bluetooth, Bluetooth low energy, and Wi-Fi, may also be employed for location detection but do not rely on time of flight. Instead such protocols typically determine location via rather unreliable received signal strength indicators (RSSI) only showing rough categories of "weak" or "strong" received signals, which grants location accuracy of much lower accuracy than UWB.

The low transmission power and wide spectrum of frequencies UWB signals are transmitted at allow for little to no interference with surrounding narrowband technologies. UWB also appears to be "invisible at noise floor", making it a good choice for coexistence with narrowband RF technologies.

Ultra-wideband, like each RF standard, offers unique characteristics and advantages that may make it a suitable option depending on individual needs, budget, facility, and specific location-based use case. The most critical differences between UWB and other technologies are its wide frequency spectrum and the level of accuracy it can provide. UWB's precision makes it an effective option, particularly in advanced indoor positioning use cases such as asset tracking scenarios, where absolute location and real-time movement is desirable.

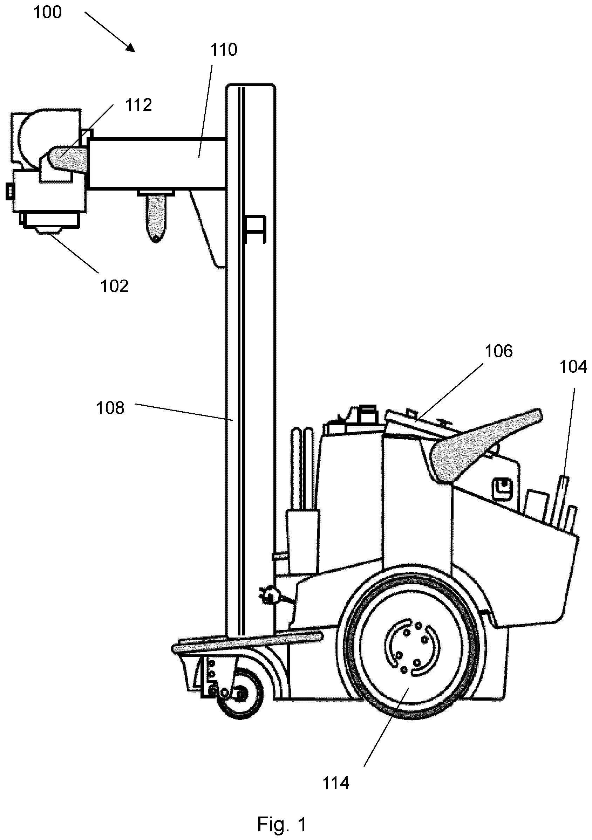

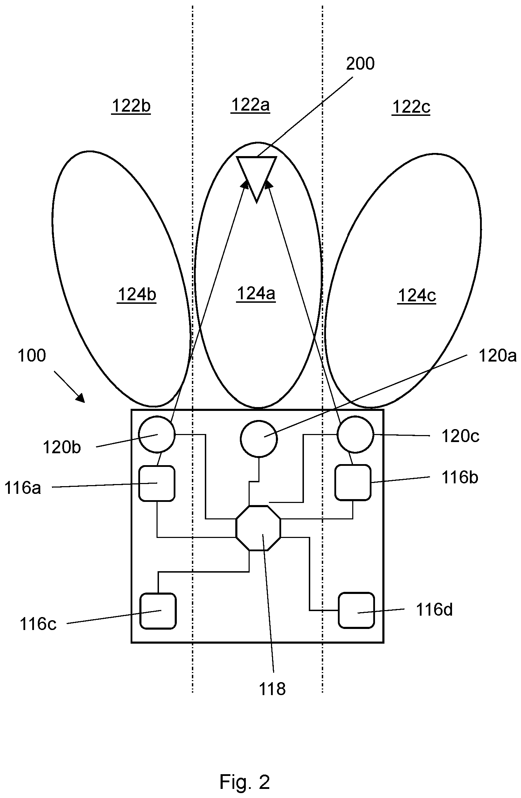

With reference to Figs. 1 to 3, there is shown a mobile radiography apparatus 100. The apparatus 100 comprises radiographic imaging means; for example,comprising a radiation generator 102 and a radiographic image sensor 104. In an example, the radiation generator 102 may be configured to generate X-rays and the image sensor 104 correspondingly configured to capture an X-ray image.

One or more elements of the radiographic imaging means 102, 104 may be coupled to suitable movement means such that one or both of the first imaging sensor 104 and radiation generator 102 may be moved relative to a target.

In the present example, the radiation generator 102 is coupled to a main body 106 of the apparatus 100 via a height adjustable neck 108, a boom arm 110, and a rotatable head 112. Meanwhile, the image sensor 104 is provided as a separate unit configured to communicate wirelessly with other components of the apparatus 100.

The apparatus 100 comprises locomotive means by which the apparatus 100 may be moved around. The locomotive means may comprise wheels, legs, tracks, and the like; the list is not exhaustive.

In the present example the locomotive means comprise at least one pair of wheels 114 which are coupled to a motor (e.g., an electric motor) to drive a forward or rearward rotation of the wheels 114. On or more other wheels 116 may be provided for stability. In the present example, rotational movement of the apparatus 100 may be controlled by driving only one of the wheels 114, or by driving both wheels in different directions.

The apparatus 100 comprises a first set of sensors 116. The set of first sensors 116 comprises at least one sensor 116a, and in some examples may comprise only one sensor 116a. In one example the first set of sensors comprises at least two sensors 116a,b to improve signal detection. In another example the first set of sensors comprises at least three sensors 116a,b,c, which has advantages for detection and triangulation algorithms which may be performed using the first set of sensors. In the present example, the first set of sensors 116 comprises four sensors, 116a-d, with each sensor being arranged proximal to a corner region of the main body 106 of the apparatus 100.

More generally, where at least four sensors 116a-d are utilised, two of the at least four sensors may be arranged towards a front of the apparatus 100, and two the at least four sensors are arranged towards a rear of the apparatus 100; similarly two of the at least four sensors may be arranged towards a left of the apparatus 100 and two to the right of the apparatus 100.

The first set of sensors are configured to receive a signal emitted by a beacon device 200. Suitably, in one example the beacon device 200 may comprise a UWB tag configured to emit a UWB signal. Accordingly, the first set of sensors may be configured to receive UWB signals. Suitably, the first set of sensors 116 may be termed a set of UWB anchors. It should however be appreciated that the disclosure is not limited thereto, and the beacon device 200 and anchors 116 may be configured to transmit and receive different RF signals such as Bluetooth, Wi-Fi, and so on (the list is not exhaustive).

The apparatus comprises a controller 118 configured to control operation of various components of the apparatus 100; for example any sensors, the radiographic imaging means, the locomotive means, and so on. Suitably the controller may comprise at least one processor and associated circuitry.

The controller 118 is configured to control a linear movement of the apparatus 100 based on the signal(s) received by the first set of sensors 116. More specifically, the controller 118 is configured to analyse each of the received signals from the sensors 118 and determine a range to the beacon device 200. Based on the determined range, the controller controls the locomotive means (e.g., wheels 114) to drive forward. The locomotive control may be based on the determined range being greater or lower than a predetermined threshold range; that is, the apparatus may be configured to maintain a predetermined separation from the beacon device 200. For example, the controller 118 may be configured to control driving motion of the apparatus to maintain a distance of three times the braking (or stopping) distance of the apparatus 100 at its current speed of motion.

In some examples, the beacon device 200 may be arranged as part of a remote controller which may comprise at least one actuator which, when moved, sends a signal to the controller 118 to control the locomotive means (e.g., wheels 114). That is, the remote controller may be used to manually control motion of the apparatus 100 if needed.

The apparatus 100 also comprises a second set of sensors 120. The set of second sensors comprises at least one sensor 120a, and in some examples may comprise only one sensor. In one example the set of second sensors 120 comprises at least two sensors to improve signal detection. In the present example example the set of second sensors 120 comprises at least three sensors. Suitably, the second set of sensors 120 may all be disposed proximal to a common edge of the apparatus 100, preferably a forward facing edge of the main body 106 (with respect to the direction of linear (forward-backward) motion of the apparatus 100)

The second set of sensors 120 are configured to detect a position of the beacon device 200. Suitably, the second set of sensors 120 may be configured to operate at a different wavelength, frequency, and on a completely different detection principle to the first set of sensors 116.

In particular, the present examples have been developed with the second set of sensors configured to detect ultrasonic waves. That is, detection of the beacon device 200 may be based on similar principles to sonar. Suitably the apparatus 100 may comprise one or more ultrasonic emitters (not shown). In another example, the second set of sensors may be configured to detect laser light of a given wavelength; suitably, the second set of sensors may be configured to detect the beacon device 200 based on lidar principles. Suitably, in such an example the apparatus 100 may comprise one or laser emitters. It will however be appreciated that the description is not limited to a specific type of detection mechanism.

Regardless of the choice of detection mechanism, it will be appreciated that, rather than detecting the position of the beacon device 200 per se, the second set of sensors may be configured to detect the position of an operator carrying the beacon device 200. Determination of the position of the operator 200, as opposed to other objects in the environment surrounding the apparatus 100, may be determined by correlating signals detected by the second set of sensors 120 with the signal(s) received by the first set of sensors 116.

Based on the determined position of the beacon device 200, the controller 118 is configured to control a rotational movement of the apparatus 100. For example, to control one of the wheels 114 to drive forward and the other wheel to drive rearward so as to rotate the apparatus 100. In this way may the controller 118 adjust an alignment of the mobile radiography apparatus 100 with respect to the beacon device.

In one example, adjustment of the orientation of the apparatus 100 may be to account for a change in direction of the operator/beacon device 200, such as taking a curved path. In another example, adjustment of the orientation may be required in order to correct a heading of the apparatus 100 back towards the operator/beacon device 200; misalignment perhaps occurring because of a surface shape over which the apparatus is moving.

In more detail, detecting the position of the beacon device 200 by the second sensors 120 may be based on dividing a detection area of the second set of sensors into zones 122. That is, the second set of sensors 120 may be arranged with a field of view pointing forward of the apparatus 100 (or more generally, away from the edge on which the sensors 120 are disposed). In one example, the detection zones 122 may correspond to the fields of view 124 of the second set of sensors 120. In another example, the detection zones may be based on sub areas of the field of view, overlapping areas of field of view, and so on.

For example, a central zone 122a may correspond to a field of view 124a of a centrally disposed sensor 120a. A left zone 122b may correspond to a field of view 124b of a sensor 120b disposed to the left of the central sensor 120a. A right zone 122c may be correspond to a field of view 124c of a sensor 120c disposed to the right of the central sensor 120a. Suitably, the beacon device 200 may be determined to be present in a zone 122a-c based on it being detected in the field of view 124a-c of the respective one of the second set of sensors 120a-c.

Zones 122 may also be defined by null areas; that is, areas outside the field of view 124 of the second set of sensors 120.

For example, where only one second sensor 120a is provided, with field of view 124a corresponding to the central zone 122a, determination of the beacon device 200 being in a left or right zone 122b,c may be based on the beacon device 200 not being detected in the central zone 122a and also toward either the left or right of the apparatus based on the signal(s) from the first set of sensors 116.

Similarly, where two sensors (left and right) 120b,c are provided, the beacon device 200 may be determined as being centrally located in zone 122a based on a null results from second sensors 120b,c and indication from first sensors 116 that the beacon device 200 is generally forward of the apparatus 100.

Suitably, the controller 118 may control alignment of the apparatus 100 based on the zone in which the locator beacon 200 is detected. That is, if the beacon 200 is detected in the central zone, then no corrective action may be needed. If the beacon is detected in the right zone 122c, then the controller 118 may control the locomotive means to rotate the apparatus to the right, until the beacon device 200 is once again detected in the central zone 122a. And vice-versa for detecting the beacon device 200 in the left zone 122b.

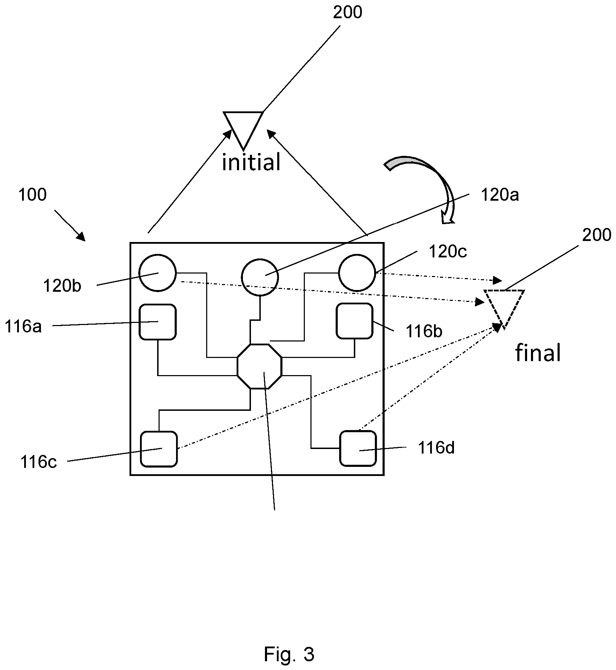

An extreme case of this realignment is shown in Fig. 3, which rather than an small adjustment in directional facing may instead be considered a turning of the apparatus 100.

Here the controller 118 receives a null result from each of the second set of sensors 120; that is, the second set of sensors 120 are not detecting the presence of the beacon device 200 within their respective fields of view. In this scenario, the controller 118 may determine a distance to the beacon device 200 from each of the first set of sensors 116. Based on the determined distances indicating that the beacon device 200 is proximal to a side, or face, of the apparatus 200 that is not the front side, then the controller 118 may control the apparatus 100 to turn to be oriented towards the beacon device 200. Once one of the second set of sensors 120 detects the beacon device 200 in its field of view, the above procedures may take over again to accurately align the apparatus 100 with the beacon 200.

While the invention has been illustrated and described in detail in the drawings and foregoing description, such illustration and description are to be considered illustrative or exemplary and not restrictive; the invention is not limited to the disclosed embodiments.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A mobile radiography apparatus (100), comprising:
a first set of sensors (116), comprising at least one sensor, configured to receive a signal emitted by a beacon device (200);
a controller (118) configured control a linear movement of the mobile radiography apparatus based on the received signal;
a second set of sensors (120), comprising at least one sensor, configured to detect a position of the beacon device;
wherein the controller is configured to control a rotational movement of the mobile radiography apparatus to adjust an alignment of the mobile radiography apparatus with respect to the beacon device, based on the detected position of the beacon device,.

2. The apparatus of claim 1, wherein the first set of sensors are configured to receive ultra-wide band radio signals.

3. The apparatus of claim 1 or 2, wherein the first set of sensors comprises at least three sensors.

4. The apparatus of claim 3, wherein the first set of sensors comprises at least four sensors, wherein two of the at least four sensors are arranged towards a front of the mobile radiography apparatus, and two the at least four sensors are arranged towards a rear of the mobile radiography apparatus.

5. The apparatus of any preceding claim, wherein the controller is further configured to, based on not detecting the beacon device by the second set of sensors, determine a distance to the beacon device from each of the first set of sensors, and control the movement of the mobile radiography apparatus to orientate a forward face of the mobile radiography apparatus towards the beacon device.

6. The apparatus of any preceding claim, wherein the second set of sensors are configured to detect ultrasound.

7. The apparatus of any preceding claim, wherein detecting the position of the beacon device comprises detecting a position of an operator carrying the beacon device.

8. The apparatus of any preceding claim, wherein detecting the position of the beacon device is based on dividing a detection area of the second set of sensors into zones (122), and wherein adjusting the alignment of the mobile radiography apparatus is based on the zone in which the locator beacon is detected.

9. The apparatus of any preceding claim, wherein each of the second set of sensors is arranged with a field of view (124) pointing forward of the mobile radiography apparatus.

10. The apparatus of claims 8 and 9, wherein separate zones are defined by separate fields of view corresponding to the second set of sensors.

11. The apparatus of any of claim 9 or 10, wherein detecting the position of the beacon device is based on detecting the beacon device within the field of view.

12. The apparatus of any preceding claim, wherein the second set of sensors comprises at least three sensors.

13. A mobile radiography system, comprising;
a remote controller comprising a beacon device (200), and
the mobile radiography apparatus (100) of any preceding claim.

14. The system of claim 13, wherein the remote controller comprises at least one actuator, and wherein movement of the actuator controls a movement of the mobile radiography apparatus.

15. The system of claim 13 or 14, wherein the beacon device is configured to emit an ultra-wide band radio signal.
